# EUROPEAN PATENT SPECIFICATION

(11) **EP 2 196 192 B1**
(45) Date of publication and mention of the grant of the patent: **13.02.2013**
(21) Application number: 08021706.0
(22) Date of filing: 15.12.2008
(51) Int. Cl.: A61K 8/898, A61Q 17/04, A61Q 5/00, A61K 8/41, A61Q 5/06

(54) **Use of aminated organopolysiloxane compounds for protecting hair and hair colour**
Verwendung von aminierten Organopolysiloxan-Verbindungen zum Schützen von Haaren und Haarfarbe
Utilisation de composés d'organopolysiloxane aminé pour protéger les cheveux et la couleur des cheveux

(43) Date of publication of application: 16.06.2010
(73) Proprietor: Kao Germany GmbH, 64297 Darmstadt (DE)
(72) Inventor: Schupp, Bettina, 64404 Bickenbach (DE); Ast, Andrea, 64283 Darmstadt (DE)
(74) Representative: Grit, Mustafa

(56) References cited:
- EP-A- 0 640 643
- EP-A- 0 697 208
- EP-A- 0 756 859
- EP-A- 0 820 757
- EP-A- 1 166 750
- EP-A- 1 166 751
- EP-A- 1 175 894
- EP-A- 1 466 581
- EP-A- 1 559 403
- EP-A- 1 982 691

## Description

The present invention relates to the use of specific aminated organopolysilioxane compounds for protecting keratin fibers form damaging effects of UV rays and sun light.

Organopolysiloxane compounds have widely been used for hair conditioning especially for improving combability, shine, manageability and other cosmetic properties of hair. Cleansing and conditioning compositions comprising organopolysiloxanes of any type are available on the market.

In order to protect hair from damaging UV rays and sunlight, so called UV filters have been proposed in the literature. Nothing is known up until now on protecting effect of a special aminated organopolysilxane compounds from UV rays and sunlight.

Inventors of the present invention have surprisingly found out that special aminated organopolysiloxane compounds are especially effective in protecting hair from UV rays and sunlight.

Such kind of aminated organopolysiloxane compounds have been disclosed in EP 640 643 A2 and several compounds are commercially available from Kao Corporation, Tokyo - Japan.

The use of aminated organopolysiloxane compounds in hair cosmetic compositions has also been known. For example, EP 756 859 A1 discloses hair styling compositions comprising the aminated organopolysiloxane compounds. Permanent waving compositions comprising the same siloxane compound are disclosed in EP 820 857 A1. Furthermore, EP 1 166 751 A1 discloses hair dyeing compositions comprising the said siloxane compound.

None of the above mentioned documents discloses the use of aminated organopolysiloxane compound for protecting hair from UV rays and sunlight.

Accordingly, the first object of the present invention is the use of a composition comprising at least one aminated organopolysiloxane compound according to general structure 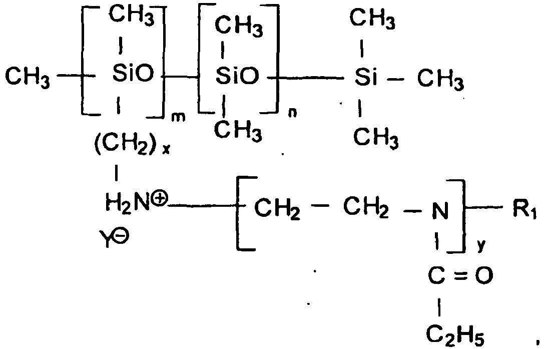 wherein m and n each are numbers from 20 to 10,000, in particular 50 to 7,000, especially 100 to 5,000, x is a number between 1 and 5, preferably 3, and y is a number from 5 to 30, R₁ is a C₁-C₁₂-alkyl or aryl group, in particular a methyl, ethyl or benzyl group, and Y⁻ is an anion, for protecting hair from UV rays and sunlight.

Further object of the present invention is process for protecting hair from UV rays and sunlight wherein a composition comprising at least one aminated organopolysiloxane compound according to general structure given above is applied onto hair and hair is optionally rinsed off.

Aminated organopolysiloxane compound can be applied in various forms onto hair such as from an alcoholic or aqueous-alcoholic solution or dispersion, gel, cream, hairspray, foam, a composition comprising at least two physically separated phases etc. Concentration of the at least one aminated organopolysiloxane compound in the compositions is in the range of 0.01 to 5%, preferably 0.05 to 3% and more preferably 0.1 to 2% and most preferably 0.1 to 1.5% by weight calculated to total composition.

For the purpose of protecting hair from UV rays and sunlight, the aminated organopolysiloxane comprising composition comprises additionally at least one UV filter. Suitable UV-absorbing substances is are ethylhexylmethoxycinnamate, 4-aminobenzoic acid and the esters and salts thereof, 2-phenyl benzimidazole-5-sulfonic acid and the alkali and amine salts thereof, 4-dimethyl aminobenzoic acid and the esters and salts thereof, cinnamic acid and the esters and salts thereof, 4-methoxycinnamic acid and the esters and salts thereof, salicylic acid and the esters and salts thereof, 2,4-dihydroxybenzophenone, 2,2',4,4'-tetrahydroxy- benzophenone, 2-hydroxy-4-methoxybenzophenone and its 5-sulfonic acid or the sodium salt thereof, 2,2'-dihydroxy-4,4'-dimethoxybenzophenone, 2-hydroxy-5-chlorobenzophenone, 2,2'-dihydroxy-4-methoxybenzophenone, 2,2'-dihydroxy-4.4'-dimethoxy-5,5'-disulfobenzo-phenone or the sodium salt thereof, 2-hydroxy-4-octyloxybenzophenone, 2-hydroxy-4-methoxy-4'-methylbenzophenone, 3-benzylidene campher, 3-(4'-sulfo)-benzylidene bornane-2-one and the salts thereof and/or 3-(4'-methyl benzylidene)-DL-campher. The amount of the UV-absorber ranges typically from about 0.01 % to 7.5%, more preferably from 0.05 % to 5 % by weight, calculated to the total composition.

In a preferred form of the invention, the conditioning composition used in the present invention can comprise one or more organic solvent such as ethanol, propanol, isopropanol, benzyl alcohol, benzyloxyethanol, ethoxydiglycol, alkylene carbonates such as ethylene carbonate and propylene carbonate, phenoxyethanol, butanol, isobutanol, cyclohexane, cyclohexanol, hexyleneglycol, ethylenecarbonate, propyleneglycol, polypropyleneglycols, ethyleneglycol monoethylether, ethylene glycol monobutyl ether, ethylene glycol monophenyl ether, 1-phenylethylalcohol, 2-phenylethylalcohol, o-methoxyphenol. The most preferred ones are ethanol, isopropanol, butanol, isobutanol, phenoxyethanol, benzylalcohol, propyleneglycol and polypropylene glycols. Concentration of organic solvents is in the range of 1 to 99.9% by weight, preferably in the range of 2 to 95%, more preferably 5 to 90% by weight calculated to total composition. It should be noted that organic solvent content of the composition depends on the type of the preparation, obviously a cream (emulsion) type of preparation comprises less organic solvent than such as a hair spray.

Any of the above mentioned compositions such as alcoholic or aqueous alcoholic solutions or dispersions, gels, creams, hairsprays, two phase compositions, etc. can comprise additional one or more of the ingredients such as surfactants, fatty alcohol, thickening agents, additional hair conditioning agents such as further silicone composunds, cationic surfactants and/or polymers, propellants, chelating agents, polyols, moisturizing agents, organic and/or inorganic acids and/or basis for adjusting pH.

Compositions used in the present invention can comprise at least one surfactant selected from anionic, nonionic, amphoteric and cationic ones. Concentration varies depending on the form of the composition in the range of 0.1 to 15%, preferably 0.25 to 10% and more preferably 0.5 to 7.5% by weight calculated to total composition.

Suitable anionic surfactants are the sulfate, sulfonate, carboxylate and alkyl phosphate type, such as C₁₀-C₁₈-alkyl sulfates, and in particular the respective ether sulfates, for example, C₁₂₋C₁₄-alkyl ether sulfate, lauryl ether sulfate, especially with 1 to 4 ethylene oxide groups in the molecule, monoglyceride (ether) sulfates, fatty acid amide sulfates obtained by ethoxylation and subsequent sulfatation of fatty acid alkanolamides, and the alkali salts thereof, as well as the salts of long-chain mono- and dialkyl phosphates.

Additional anionic surfactants are α-olefin sulfonates or the salts thereof, and in particular alkali salts of sulfosuccinic acid semiesters, for example, the disodium salt of monooctyl sulfosuccinate and alkali salts of long-chain monoalkyl ethoxysulfosuccinates.

Suitable surfactants of the carboxylate type are alkyl polyether carboxylic acids and the salts thereof, which can optionally be hydroxyalkyl-substituted, and as well as alkyl amido polyether carboxylic acids especially the ones with 1 to 10 ethoxy units. Also useful are C₈-C₂₀-acyl isethionates, alone or in admixture with other anionic surfactants, as well as sulfofatty acids and the esters thereof. Further suitable anionic surfactants are also C₈-C₂₂-acyl aminocarboxylic acids or the water-soluble salts thereof, such as N-lauroyl glutamate, in particular as sodium salt, N-lauroyl sarcosinate, N-C₁₂-C₁₈-acyl asparaginic acid, N-myristoyl sarcosinate, N-oleoyl sarcosinate, N-lauroyl methylalanine, N-lauroyl lysine and N-lauroyl aminopropyl glycine, preferably in form of the water-soluble alkali or ammonium, in particular the sodium salts thereof.

Suitable nonionic surfactants such as alkyl polyglucosides with 1 to 5 saccharide groups, long-chain fatty acid mono- and dialkanolamides, such as coco fatty acid monoethanolamide and myristic fatty acid monoethanolamide, the various sorbitan esters, such as polyethylene glycol sorbitan stearic acid ester, fatty acid polyglycol esters or poly-condensates of ethyleneoxide and propyleneoxide, as well as fatty alcohol ethoxylates such as C₁₀-C₂₂-fatty alcohol ethoxylates with average degree of ethoxylation between 1 and 60, known by the generic terms "Laureth", "Myristeth", "Oleth", "Ceteth", "Deceth", "Steareth" and "Ceteareth" according to the CTFA nomenclature, including addition of the number of ethylene oxide molecules, e.g., "Laureth-16":
Suitable amphoteric surfactants are such as betaines especially alkyl betaines, fatty acid amidoalkyl betaines and sulfobetaines, for example, lauryl hydroxysulfobetaine; long-chain alkyl amino acids, such as cocoaminoacetate, cocoaminopropionate and sodium cocoamphopropionate and -acetate have also proven suitable.

Suitable cationic surfactants are according to the general formula 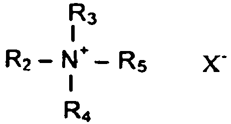 where R₂ is a saturated or unsaturated, branched or non-branched alkyl chain with 8-24 C atoms or

R₆ CO NH (CH₂)ₙ

where R₆ is saturated or unsaturated, branched or non-branched alkyl chain with 7-21 C atoms and n has value of 1 - 4, or

R₇ CO O (CH₂)ₙ

where R₇ is saturated or unsaturated, branched or non-branched alkyl chain with 7-21 C atoms and n has value of 1 - 4, and
R₃ is unsaturated or saturated, branched or non-branched alkyl chain with 1 - 24 C atoms or

R₆ CO NH (CH₂)ₙ

or

R₇ CO O (CH₂)ₙ

where R₆, R₇ and n are same as above,
R₄ and R₅ are lower alkyl chain with 1 to 4 carbon atoms which may be substituted with one or more hydroxyl group, and X is anion such as chloride, bromide, methosulfate.

Typical examples of those ingredients are cetyltrimethyl ammonium chloride, steartrimonium chloride, behentrimonium chloride, stearamidopropyl trimonuim chloride, dioleoylethyl dimethyl ammonium methosulfate, dioleoylethyl hydroxyethylmonium methosulfate.

Amido amines may as well be used as a conditioning cationic surfactant in the compositions of the present invention. Typical non-limiting example is stearamidopropyl dimethyl amine known with a trade name Tego Amid S18 from Degussa and Lexamine S13 from Inolex.

It should be noted that cationic surfactants are at the same time conditioners and also especially mono alkyl cationic surfactants are preferred emulsifiers when emulsion type of composition is prepared.

Compositions used in the present invention are suitable for either rinse off or leave in applications. Especially preferred use is as leave-in usage wherein after application of special aminated organopolysiloxane comprising composition it is not rinsed off from hair.

In another preferred from of the present invention, the compositions used in the present invention comprise at least one special aminated organosiloxane compound and comprise hair-conditioning agents in any type of composition. Conditioning agents can be selected from oily substances, non-ionic substances, cationic amphiphilic ingredients, cationic polymers or their mixtures.

Oily substances are selected from such as silicone oils, eithervolatile or nonvolatile, natural and synthetic oils. Among silicone oils those can be added to the compositions include dimethicone, dimethiconol, polydimethylsiloxane, DC fluid ranges from Dow Corning, arylated silicones such as phenyl methicone, phenyl trimethicone, diphenyl dimethicone, diphenylsiloxy phenyl trimethicone, tetramethyl tetraphenyl trisiloxane, triphenyl trimethicone, and trimethyl pentaphenyl trisiloxan, natural oils such as olive oil, almond oil, avocado oil, wheatgerm oil, ricinus oil and the synthetic oils, such as mineral oil, isopropyl myristate, palmitate, stearate and isostearate, oleyl oleate, isocetyl stearate, hexyl laurate, dibutyl adipate, dioctyl adipate, myristyl myristate and oleyl erucate.

Concentration of one or more oily substances is in the range of 0.01 to 35%, preferably 0.05 to 30%, and more preferably 0.1 to 25% by weight calculated to total composition. The concentrations referred here are total concentration of total concentration of all oily substances may be present in the composition.

Non-ionic conditioning agents may be polyols such as glycerin, glycol and derivatives, polyethyleneglycoles known with trade names Carbowax PEG from Union Carbide and Polyox WSR range from Amerchol, polyglycerin, polyethyleneglycol mono or di fatty acid esters having general formula

R₈ CO (O CH₂ CH₂)ₙ OH

or

R₈ CO (O CH₂CH₂)ₙ O OC R₉

where R₈ and R₉ are independent from each other saturated, unsaturated or branched or non-branched alkyl chain with 7 to 21 C atoms and n is typically 2 - 100.

In one of the preferred forms of the present invention, compositions used comprise at least one cationic polymer as conditioning agent. Suitable cationic polymers are those of best known with their CTFA category name Polyquaternium. Typical examples of those are Polyquaternium 1, Polyquaternium 2, Polyquaternium 4, Polyquaternium 5, Polyquaternium 6, Polyquaternium 7, Polyquaternium 8, Polyquaternium 9, Polyquaternium 10, Polyquaternium 11, Polyquaternium 12, Polyquaternium 13, Polyquaternium 14, Polyquaternium 15, Polyquaternium 16, Polyquaternium 17, Polyquaternium 18, Polyquaternium 19, Polyquaternium 20, Polyquaternium 22, Polyquaternium 24, Polyquaternium 27, Polyquaternium 28, Polyquaternium 29, Polyquaternium 30, Polyquaternium 31, Polyquaternium 32, Polyquaternium 33, Polyquaternium 34, Polyquaternium 35 and Polyquaternium 36, Polyquaternium-37, Polyquaternium 39, Polyquaternium 42, Polyquaternium 43, Polyquaternium 44, Polyquaternium 45, Polyquaternium 46, Polyquaternium 47, Polyquaternium 48, Polyquaternium-49, Polyquaternium 50, Polyquaternium 51, Polyquaternium 52, Polyquaternium 53, Polyquaternium 54, Polyquaternium 55, Polyquaternium 56, Polyquaternium 57, Polyquaternium 58, Polyquaternium 59, Polyquaternium 60, Polyquaternium 61, Polyquaternium 62, Polyquaternium 63, Polyquaternium 64, Polyquaternium 65, Polyquaternium 66, Polyquaternium 67, Polyquaternium 68, Polyquaternium 69, Polyquaternium-70, Polyquaternium 71, Polyquaternium 72, Polyquaternium 73, Polyquaternium 74, Polyquaternium 75, Polyquaternium 76, Polyquaternium 77, Polyquaternium 78, Polyquaternium-79, Polyquaternium 80, Polyquaternium 81, Polyquaternium 82, Polyquaternium 83, Polyquaternium 84, Polyquaternium 85, Polyquaternium 86 and Polyquaternium 87 as well as silicone quaternium-1, silicone quaternium-2, silicone quaternium-2 panthenol succinate, silicone quaternium-3, silicone quaternium-4, silicone quaternium-5, silicone quaternium-6, silicone quaternium-7, silicone quaternium-8, silicone quaternium-9, silicone quaternium-10, silicone quaternium-11, silicone quaternium-12, silicone quaternium-15, silicone quaternium-16, silicone quaternium-16/Glycidoxy Dimethicone Crosspolymer, silicone quaternium-17, silicone quaternium-18, silicone quaternium-20 and silicone quaternium-21.

It has further been found out that especially those of cationic cellulose type polymers known as Polymer JR type from Amerchol such as Polyquaternium 10 or cationic galactomannans such as cationic guar gum known with trade name Jaguar from Rhône-Poulenc which are chemically for example Guar hydroxypropyl trimonium chloride and cationic tara gum an its derivatives known with INCI name Caesalpinia spinosa hydroxypropyltrimonium chloride, are especially suitable ones. Furthermore, chitosan and chitin can also be included in the compositions as cationic natural polymers.

Compositions used in the present invention may also comprise further conditioning substances such as protein hydrolyzates and polypeptides, e.g., keratin hydrolyzates, collagen hydrolyzates of the type "Nutrilan^{R}" or elastin hydrolyzates, as well as also in particular plant protein hydrolyzates, optionally, cationized protein hydrolyzates, e.g., "Gluadin^{R}"

Typical concentration range for any of those conditioners of cationic polymers, silicon oil and derivatives can be 0.01 - 10% by weight, preferably 0.01 - 7.5% by weight, more preferably 0.05 - 5% and most preferably 0.1 - 3% by weight calculated to the total composition.

The composition used in the present invention can comprise at least one polyphenol. With the word polyphenol it is meant that an organic molecule with at least 2 hydroxyl groups in its molecule

In the preferred form of the invention, at least one polyphenol or mixture of polyhenols is included into the composition used in the present invention from a natural plant extract. In principal any natural plant extract rich of polyphenols is suitable within the meaning of the present invention. The extracts are liquid extracts and prepared by mixing plant parts such as leaves, fruits, blossoms and roots with a solvent such as water, alcohol, propyleneglycol or mixture of more than one solvent and incubating for certain period of time and filtrating the undissolved plant parts. Suitable aqueous (e.g. steam-distilled) alcoholic or hydro-alcoholic plant extracts known per se are in particular aloe, pineapple, artichoke, arnica, avocado, valerian, bamboo, henbane, birch, stinging nettle, echinacea, ivy, wild angelica, gentian, ferns, pine needles, silver weed, ginseng, broom, oat, rose hip, hamamelis, hay flowers, elderberry, hop, coltsfoot, currants, chamomile, carrots, chestnuts, clover, burr root, cocoanut, cornflower, lime blossom, lily of the valley, marine algae, balm, mistletoe, passion flower, ratanhia, marigold, rosemary, horse chestnut, pink hawthorn, sage, horsetail, yarrow, primrose, nettle, thyme, walnut, wine leaves, white hawthorn, etc. Suitable trade products are, for example, the various "Extrapon®" products, "Herbasol^{R} ", "Sedaplant^{R}" and "Hexaplant^{R}". Extracts and the preparation thereof are also described in "Hagers Handbuch der pharmazeutischen Praxis", 4^{th} Ed. Preferred plant extracts are prepared from Vitis vinifera, Malus domestica, Camelia sinensis, Juglans regia Ribes Uva-Crispa, Ribes nigrum, Ribes rubrum and Punica granatum. The above mentioned extracts may also be available in the powder form and such are also suitable within the meaning of the present invention.

The polyphenol comprising extracts are included into the compositions of the present invention at a concentration of 0.001 to 10%, preferably0.005 to 7.5%, more preferably 0.01 to 5% and most preferably 0.05 to 2.5% by weight, calculated to total composition based on dry matter of the extract.

Especially the emulsion type of compositions comprise additionally at least one fatty alcohol of the following formula

R₁₅-OH

where R₁₅ is a saturated or unsaturated, branched or non-branched fatty acyl chain with 8 - 24 C atoms. Concentration of fatty alcohols is usually less than 20%, preferably in the range of 1 to 15% by weight calculated to total composition. Typical examples to the most useful fatty alcohols are myristyl alcohol, palmityl alcohol, cetyl alcohol, stearyl alcohol, behenyl alcohol and their mixtures. As a mixed fatty alcohol the mostly used one is the cetearyl alcohol as well preferred in the compositions of the present invention.

Compositions used in the present invention can comprise moisturizers, chelating agents, preservatives and fragrance. The moisturizing agents are selected from panthenol, polyols, such as glycerol, polyethylene glycols with molecular weight 200 to 20,000. The moisturizing ingredients can be included compositions at a concentration range of 0.01 - 2.5% by weight calculated to the total composition.

The sequestering agents are selected from polycarboxy acids. The preferred one is ethylene diamine tetraacetic acid, EDTA. Typical useful concentration range for sequestering agents is of 0.01 - 2.5% by weight calculated to the total composition.

Solubilizers may be added to the compositions used in the present invention, especially when oily substances are chosen conditioning agents and fragrance oils with highly lipophilic properties. Typical solubilizers may be hydrogenated castor oil known with the trade mark Cremophor RO series from BASF. It should be noted that as well the surfactant mixture can be a good solubilizer for fragrance oils. Typical concentration of the solubilizers can be in the range of 0.01 - 2% by weight, preferably 0.1 - 1% by weight, calculated to total composition.

Further conditioning additives are hair conditioning and/or styling polymers. These may be nonionic polymers, preferably alcohol- and/or water-soluble vinyl pyrrolidone polymers, such as a vinyl pyrrolidone homopolymers or copolymers, in particular with vinyl acetate. Useful vinyl pyrrolidone polymers are, e.g., those known by the trade name "Luviskol®", for example, the homopolymers "Luviskol® K 30, K 60 and K 90", as well as the water-or alcohol-soluble copolymers from vinyl pyrrolidone and vinyl acetate, distributed by BASF AG under the trade name "Luviskol® VA 55 respectively VA 64". Further possible nonionic polymers are vinyl pyrrolidone/vinyl acetate/vinyl propionate copolymers such as "Luviskol® VAP 343", vinyl pyrrolidone/(meth)acrylic acid ester copolymers, as well as chitosan derivatives.

Amphoteric polymers are found to be useful in the conditioning composition used in the present invention. They are incorporated alone or in admixture with at least one additional cationic, nonionic or anionic polymer, particularly copolymers of N-octyl acrylamide, (meth)acrylic acid and tert.-butyl aminoethyl methacrylate of the type "Amphomer®"; copolymers from methacryloylethyl betaine and alkyl methacrylates of the type "Yukaformer®", e.g., the butyl methacrylate copolymer "YukaformerO Am75"; copolymers from monomers containing carboxyl groups and sulfonic groups, e.g., (meth)acrylic acid and itaconic acid, with monomers such as mono- or dialkyl amino alkyl(meth)acrylates or mono- or dialkyl aminoalkyl (meth)acrylamides containing basic groups, in particular amino groups; copolymers from N-octyl acrylamide, methyl methacrylate, hydroxypropyl methacrylate, N-tert.-butyl aminoethyl methacrylate and acrylic acid, as well as the copolymers known from US-A 3,927,199, are applicable.

The compositions used in the present invention can be in the form of aerosol spray and foam and comprise propellant gas. The suitable propellant gasses are carbondioxide, dimethylether and alkanes such as butane, propane, isobutane or their mixtures.

The pH of the compositions is suitably between 2 and 8 and preferably in the range of 2.5 to 7.5, more preferably 3 to 7 and most preferably 3.5 to 6.5.

In principal pH of the compositions used in the present invention can be adjusted with any organic and/or inorganic acids or their mixture. Some of them to mention are phosphoric acid, hydrochloric acid as the inorganic ones and to the organic acids the well known citric acid and lactic acid, glycolic acid, glyceric acid, malic acid and tartaric acid and of the dicarboxylic acids are malonic acid, succinic acid, glutaric acid, adipic acid, maleic acid, fumaric acid and phtalic acid.

The compositions used in the present invention can further comprise at least one thickening and/or gelling agent. Suitable ones are cellulose and its derivatives such as methyl cellulose, ethyl cellulose and hydroxyethylcellulose, xanthan gum, guar gum and as a synthetic one acrylate polymers suc as carbomer andor alkyl acrylates and their derivatives. Concentration of thickening and/or gelling agent is, depending on the desired consistency, is in the range of 0.1 to 2.5% by weight calculated to total composition.

The following examples are to illustrate the invention, but not to limit.

### Example 1

| | % by weight |
|---|---|
| OS- 96* | 0.5 |
| Ethanol | 99.5 |

| | |
|---|---|
| *is an aminated organopolysiloxane compound according to above given general structure available from Kao Corporation. | |

For testing the effect of the above composition, a hair tress was used which was colored with a commercially available oxidative coloring composition into red-copper color direction and the above composition was sprayed onto dry hair and hair was dried.

For testing UV and sunlight protection effect, hair was placed in artificial UV-sunlight equipment (Sunset Machine model Sunset CPS supplied by Atlas Material Testing Solution with a light emission energy of the equipment is 765 W/m² ± 10%) and exposed to artificial sunlight for 72 hrs. Before and after sunlight exposure L, a and b values were measured with a laboratory colorimeter and color differences as expressed by ΔE values were calculated using the well known equation. For comparative purpose ethanol was sprayed onto hair and treated in the same way as described above. Following results were obtained.

**Table I: Results of the sun exposure test**

| | ΔE value |
|---|---|
| Inventive composition | 6.16 |
| Comparative composition | 11.78 |

From the above results, it is clear that OS-96 is effectively protects hair color from sunlight exposure.

Similar results were observed with the following examples.

### Example 2

| | % by weight |
|---|---|
| OS- 96* | 0.5 |
| Benzophenone-3 | 0.5 |
| Ethanol | 99.0 |

The above composition was applied onto hair by spraying

### Example 3

| | % by weight |
|---|---|
| OS- 96* | 0.5 |
| Cetrimonium chloride | 2.5 |
| Ethylhexyl methoxy cinnamate | 0.2 |
| Ethanol | 47.0 |
| Water | to 100 |

Above composition is a solution and applied onto hair by spraying with a pump spray.

### Example 4

| | % by weight |
|---|---|
| OS- 96* | 0.5 |
| Ethylhexyl methoxy cinnamate | 0.2 |
| Carbopol Aqua SF 1 | 1.0 |
| Sodium hydroxide | q.s. to pH 6.5 |
| Ethanol | 40.0 |
| Water | to 100 |

Above composition is a gel and applied onto hair by hand.

### Example 5

| | % by weight |
|---|---|
| OS- 96* | 0.5 |
| Benzophenone-3 | 0.2 |
| Cetrimonium chloride | 2.5 |
| Hydroxyethylcellulose | 1.0 |
| Ethanol | 30.0 |
| Water | to 100 |

Above composition is a gel and applied onto hair by hand.

### Example 6

| | % by weight |
|---|---|
| OS- 88* | 0.5 |
| Ethylhexyl methoxy cinnamate | 0.2 |
| Cetrimonium chloride | 2.5 |
| Polyquaternium-10 | 0.5 |
| Hydoxyethylcellulose | 0.8 |
| Ethanol | 30.0 |
| Water | to 100 |

| | |
|---|---|
| *is an aminated organopolysiloxane compound according to above given general structure available from Kao Corporation. | |

Above composition is a gel and applied onto hair by hand.

### Example 7

| | % by weight |
|---|---|
| OS- 88* | 0.5 |
| Cremophor RO-60 | 2.0 |
| Ethylhexyl methoxy cinnamate | 0.2 |
| Ethanol | 47.0 |
| Water | to 80 |
| Dimethylether | 5 |
| Propane/butane | 15 |

Above composition is a hair spray and confectioned in an aerosol can and sprayed onto hair.

### Example 8

| | % by weight |
|---|---|
| OS- 88* | 0.5 |
| Almond oil | 0.2 |
| Cetearyl alcohol | 10.0 |
| Cetrimonium chloride | 2.0 |
| Benzophenone-3 | 0.5 |
| Propylene glycol | 3.0 |
| Ethanol | 10.0 |
| Citric acid/Sodium hydroxide | q.s. to pH 4.5 |
| Fragrance, preservative | q.s. |
| Water | to 80 |

Above composition is an emulsion and applied onto hair by hand.

### Example 9

| | % by weight |
|---|---|
| **Oil phase** | |
| OS- 88* | 0.5 |
| Phenyltrimethicone | 0.3 |
| Dimethicone 1cSt | 30 |

| **Water phase** | |
|---|---|
| Benzophenone-4 | 0.3 |
| Cetrimonium chloride | 0.4 |
| Polyquaternium-11 | 0.2 |
| Ethanol | 10.0 |
| Citric acid/Sodium hydroxide | q.s. to pH 5.0 |
| Fragrance, preservative | q.s. |
| Water | to 100 |

Above composition is a two phase composition which appears as two separated optically separated phases and applied onto hair after shaking to homogeneity with a pump spray. After release of shaking, the composition returns into two optically separated phases in a relatively short period of time.

## Claims

1. Use of a composition comprising at least one aminated organopolysiloxane compound according to general structure 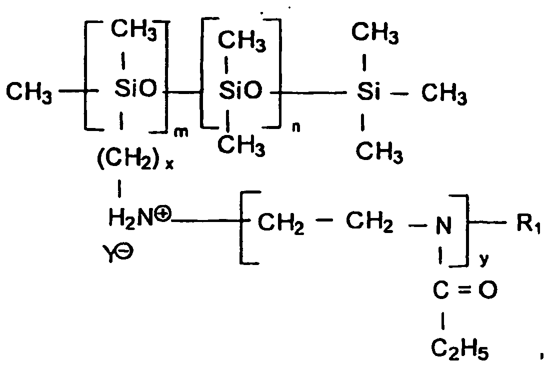 wherein m and n each are numbers from 20 to 10,000, in particular 50 to 7,000, especially 100 to 5,000, x is a number between 1 and 5, preferably 3, and y is a number from 5 to 30, R₁ is a C₁-C₁₂-alkyl or aryl group, in particular a methyl, ethyl or benzyl group, and Y⁻ is an anion, for protecting hair from UV rays and sunlight.

2. Use according to claim 1 **characterized in that** composition comprises at least one UV filter.

3. Use according to claims 1 and 2 **characterized in that** composition comprises at least one organic solvent, preferably selected from ethanol, isopropanol, butanol, isobutanol, phenoxyethanol, benzylalcohol, propyleneglycol and polypropylene glycols.

4. Use according to any of the preceding claims **characterized in that** composition comprises at least one surfactant selected from anionic, nonionic, amphoteric and cationic surfactants.

5. Use according any of the preceding claims **characterized in that** composition comprises at least one conditioning agent, preferably selected from oily substances, nonionic substances, cationic amphiphilic ingredients and cationic polymers or their mixtures.

6. Use according to claim 5 **characterised in that** at least one conditioning agent is a cationic surfactant according to general structure 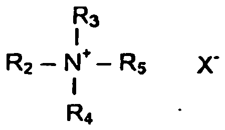 where R₂ is a saturated or unsaturated, branched or non-branched alkyl chain with 8-24 C atoms or
R₆ CO NH (CH₂)ₙ
where R₆ is saturated or unsaturated, branched or non-branched alkyl chain with 7-21 C atoms and n has value of 1 - 4,
or
R₇ CO O (CH₂)ₙ
where R₇ is saturated or unsaturated, branched or non-branched alkyl chain with 7-21 C atoms and n has value of 1 - 4, and
R₃ is unsaturated or saturated, branched or non-branched alkyl chain with 1 - 24 C atoms or
R₆ CO NH (CH₂)ₙ
or
R₇ CO O (CH₂)ₙ
where R₆ , R₇ and n are same as above,
R₄ and R₅ are lower alkyl chain with 1 to 4 carbon atoms which may be substituted with one or more hydroxyl group, and X is anion such as chloride, bromide, methosulfate.

7. Use according to claim 5 **characterized in that** oily conditioning agent is a silicone oil.

8. Use according to any of the preceding claims **characterized in that** composition comprises at least one thickening agent.

9. Use according to any of the preceding claims **characterized in that** composition comprises at least one fatty alcohol according to general formula
R₁₅-OH
where R₁₅ is a saturated or unsaturated, branched or non-branched fatty acyl chain with 8 - 24 C atoms.

10. Use according to any of the preceding claims **characterized in that** composition comprises at least one propellant.

11. Use according to any of the preceding claims **characterized in that** composition comprises at least one hair styling polymer.

12. Process for protecting hair from UV rays and sunlight wherein a composition according to claims 1 to 11 is applied onto hair and hair is optionally rinsed off.

## Patentansprüche

1. Verwendung einer Zusammensetzung die mindestens eine aminierte Organopolysiloxan- Verbindung entsprechend der allgemeinen Formel enthält, 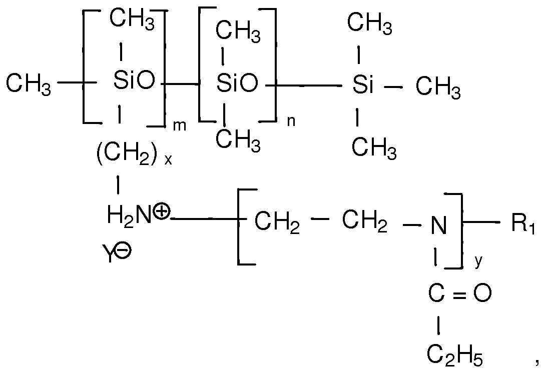 worin m und n jeweils Zahlen von 20 bis 10.000, besonders 50 bis 7.000 insbesondere 100 bis 5.000 sind, x eine Zahl zwischen 1 und 5, vorzugsweise 3 ist, und y eine Zahl von 5 bis 30 ist, R₁ eine C₁-C₁₂-Alkyl - oder Aryl- Gruppe, besonders eine Methyl-, Ethyl- oder Benzyl- Gruppe ist, und Y- ein Anion ist, um das Haar vor UV- Strahlung und Sonnenlicht zu schützen.

2. Verwendung nach Anspruch 1, **dadurch gekennzeichnet, dass** die Zusammensetzung mindestens einen UV- Filter enthält.

3. Verwendung nach den Ansprüchen 1 und 2, **dadurch gekennzeichnet, dass** die Zusammensetzung mindestens ein organisches Lösungsmittel enthält, vorzugsweise ausgewählt aus Ethanol, Isopropanol, Butanol, Isobutanol, Phenoxyethanol, Benzylalkohol, Propylenglykol und Polypropylenglykolen.

4. Verwendung nach einem der vorhergehenden Ansprüche, **dadurch gekennzeichnet, dass** die Zusammensetzung mindestens ein Tensid, ausgewählt aus anionischen, nicht ionischen, amphoteren und kationischen Tensiden enthält.

5. Verwendung nach einem der vorhergehenden Ansprüche, **dadurch gekennzeichnet, dass** die Zusammensetzung mindestens ein Konditionierungsmittel enthält, vorzugsweise ausgewählt aus öligen Substanzen, nicht ionischen Substanzen, kationisch amphiphilischen Inhaltsstoffen und kationischen Polymeren oder ihren Mischungen.

6. Verwendung nach Anspruch 5, **dadurch gekennzeichnet, dass** mindestens ein Konditionierungsmittel ein kationisches Tensid nach der allgemeinen Struktur ist 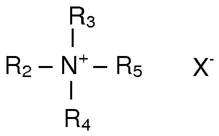 wo R₂ eine gesättigte oder ungesättigte, verzweigte oder nicht verzweigte Alkylkette mit 8 - 24 C-Atomen ist, oder
R₆ CO NH (CH₂)ₙ
wo R₆ eine gesättigte oder ungesättigte, verzweigte oder nicht verzweigte Alkylkette mit ^{7 - 21} C- Atomen ist und n einen Wert von 1 - 4 hat,
oder
R₇ CO O (CH₂)ₙ
wo R₇ eine gesättigte oder ungesättigte, verzweigte oder nicht verzweigte Alkylkette mit 7 - 21 C- Atomen ist und n einen Wert von 1 - 4 hat, und R₃ eine ungesättigte oder gesättigte, verzweigte oder nicht verzweigte Alkylkette mit 1 - 24 C- Atomen ist oder
R₆ CO NH (CH₂)ₙ
oder
R₇ CO O (CH₂)ₙ
wo R₆, R₇ und n gleich wie oben sind,
R₄ und R₅ niedrige Alkylketten mit 1 bis 4 Kohlenstoffatomen sind, die mit einer oder mehreren Hydroxygruppen substituiert sein können, und X ein Anion wie Chlorid, Bromid oder Methosulfat ist.

7. Verwendung nach Anspruch 5, **dadurch gekennzeichnet, dass** das ölige Konditionierungsmittel ein Silikonöl ist.

8. Verwendung nach einem der vorhergehenden Ansprüche, **dadurch gekennzeichnet, dass** die Zusammensetzung mindestens ein Verdickungsmittel enthält.

9. Verwendung nach einem der vorhergehenden Ansprüche, **dadurch gekennzeichnet, dass** die Zusammensetzung mindestens einen Fettalkohol entsprechend der allgemeinen Formel
R₁₅-OH
enthält,
worin R₁₅ eine gesättigte oder ungesättigte, verzweigte oder nicht verzweigte Fettsäure- Acykette mit 8 - 24 C- Atomen ist.

10. Verwendung nach einem der vorhergehenden Ansprüche, **dadurch gekennzeichnet, dass** die Zusammensetzung mindestens ein Treibmittel enthält.

11. Verwendung nach einem der vorhergehenden Ansprüche, **dadurch gekennzeichnet, dass** die Zusammensetzung mindestens ein Haarstyling-Polymer enthält.

12. Verfahren zum Schutz der Haare vor UV- Strahlen und Sonnenlicht, worin eine Zusammensetzung nach den Ansprüchen 1 bis 11 auf das Haar aufgetragen wird und das Haar optional ausgespült wird.

## Revendications

1. Utilisation d'une composition comprenant au moins un composé organopolysiloxane aminé selon la structure générale 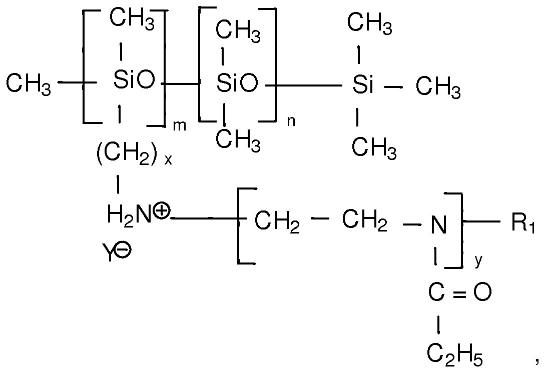 où m et n représentent chacun des nombres de 20 à 10.000, en particulier de 50 à 7.000, surtout de 100 à 5.000, x représente un nombre entre 1 et 5, de préférence 3, et y représente un nombre de 5 à 30, R₁ représente un groupe C₁-C₁₂-alkyle ou aryle, en particulier un groupe de méthyle, d'éthyle ou de benzyle, et Y- représente un anion, pour protéger les cheveux des rayons ultraviolets et de la lumière du soleil.

2. Utilisation selon la revendication 1, **caractérisée en ce que** la composition comprend au moins un filtre ultraviolet.

3. Utilisation selon les revendications 1 et 2, **caractérisée en ce que** la composition comprend au moins un solvant organique, choisi de préférence parmi éthanol, isopropanol, butanol, isobutanol, phénoxyéthanol, alcool benzylique, propylèneglycol et polypropylèneglycols.

4. Utilisation selon une quelconque des revendications précédentes, **caractérisée en ce que** la composition comprend au moins un tensio-actif choisi parmi des tensio-actifs anioniques, non-ioniques, amphotères et cationiques.

5. Utilisation selon une quelconque des revendications précédentes, **caractérisée en ce que** la composition comprend au moins un agent de conditionnement, de préférence choisi parmi des substances huileuses, des substances non-ioniques, des ingrédients amphiphiles cationiques et des polymères cationiques ou leurs mélanges.

6. Utilisation selon la revendication 5, **caractérisée en ce que** au moins un agent de conditionnement est un tensio-actif cationique selon la structure générale 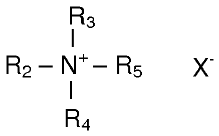 où R₂ représente une chaîne alkyle saturée ou non-saturée, ramifiée ou non ramifiée avec 8 à 24 atomes de C ou
R₆ CO NH (CH₂)ₙ
où R₆ représente une chaîne alkyle saturée ou non-saturée, ramifiée ou non ramifiée avec 7 à 21 atomes de C et n a une valeur de 1 à 4,
ou
R₇ CO O (CH₂)ₙ
où R₇ représente une chaîne alkyle saturée ou non-saturée, ramifiée ou non ramifiée avec 7 à 21 atomes de C et n a une valeur de 1 à 4, et
R₃ représente une chaîne alkyle saturée ou non-saturée, ramifiée ou non ramifiée avec 7 à 24 atomes de C ou
R₆ CO NH (CH₂)ₙ
ou
R₇ CO O (CH₂)ₙ
où R₆, R₇ et n sont les mêmes que ci-dessus,
R₄ et R₅ représentent une chaîne alkyle inférieure avec 1 à 4 atomes de carbone pouvant être remplacée par un ou plusieurs groupes hydroxyles, et X représente un anion tel que chlorure, bromure, méthosulfate.

7. Utilisation selon la revendication 5, **caractérisée en ce que** l'agent de conditionnement huileux est une huile silicone.

8. Utilisation selon une quelconque des revendications précédentes, **caractérisée en ce que** la composition comprend au moins un agent épaississant.

9. Utilisation selon une quelconque des revendications précédentes, **caractérisée en ce que** la composition comprend au moins un alcool gras selon la formule générale
R₁₅-OH
où R₁₅ représente une chaîne d'acyle gras, saturée ou non-saturée, ramifiée ou non ramifiée avec 8 à 24 atomes de C.

10. Utilisation selon une quelconque des revendications précédentes, **caractérisée en ce que** la composition comprend au moins un agent propulseur.

11. Utilisation selon une quelconque des revendications précédentes, **caractérisée en ce que** la composition comprend au moins un polymère pour le coiffage des cheveux.

12. Procédé pour protéger les cheveux des rayons ultraviolets et de la lumière du soleil, suivant lequel une composition selon les revendications 1 à 11 1 est appliquée sur les cheveux et les cheveux sont rincés facultativement.
